# EUROPEAN PATENT APPLICATION

(11) **EP 4 768 573 A1**
(43) Date of publication of application: **01.07.2026**
(21) Application number: 24307292.3
(22) Date of filing: 24.12.2024
(51) Int. Cl.: C12N 5/0783

(54) **METHOD FOR PRODUCING GAMMA DELTA T CELLS**

(71) Applicant: Université de Lorraine, 54052 Nancy Cedex (FR); Centre National de la Recherche Scientifique, 75016 Paris (FR); Centre Hospitalier Régional Universitaire de Nancy, 54035 Nancy Cedex (FR)
(72) Inventor: GUEDON, Emmanuel, 54600 VILLERS-LÈS-NANCY (FR); DECOT, Véronique, 88330 DOMÈVRE-SUR-DURBION (FR); SCHIAVI, Jessica, 54000 NANCY (FR); GAUTHIER, Mélanie, 54000 NANCY (FR); HERZOG, Marine, 54000 NANCY (FR); PORTE, Mélissa, 54000 NANCY (FR)
(74) Representative: Gevers & Orès

(57) **Abstract**

The present invention relates to a method for producing gamma delta (γδ) T cells. The present invention also relates to the cells obtained by said method and said cells for their use as a medicament and in an immunotherapy, preferably in the treatment or prevention of a cancer.

## Description

The present invention relates to a method for producing gamma delta (γδ) T cells. The present invention also relates to the cells obtained by this method and said cells for their use as a medicament, preferably in an immunotherapy, more preferably in the treatment or prevention of a cancer.

Cellular immunotherapy is part of the therapeutic arsenal that can be used to treat cancers and hematological malignancies such as leukemia. It consists of isolating immune cells with antitumor activity of autologous origin (from the patient) or allogeneic origin (from a donor) and reinjecting them into the patient either in native form or after genetic modification. The cellular immunotherapies most widely used currently include T lymphocytes (T cells) and Natural Killer (NK) cells. For example, injections of native T cells from donors are regularly performed following allogeneic hematopoietic stem cell (HSC) transplantation when the patient's disease relapses (Abbi et al., Bone Marrow transplant., 48:357-362, 2013). However, such injections are often accompanied by the risk of triggering a graft-versus-host (GvH) reaction. This complication results in an attack of the recipient's epithelial tissues by the donor's T cells and is mainly due to a subpopulation of T cells having a T cell receptor alpha beta (TCR αβ). This complication is very frequent after an allogeneic HSC transplantation and can, in the most severe forms, be life-threatening for the patient.

In recent years, T cells have also been used as a platform for the production of chimeric antigenic receptor-T cells (or CAR-T cells). These are cells genetically modified to express a specific receptor for an antigen expressed by tumors and they are particularly appropriate for the treatment malignant blood diseases. Because of this genetic modification, CAR-T cells have specificity against the tumor and a more targeted cytotoxic effect. Today, only autologous CAR-T cells from the patient are used because allogeneic CAR-T cells, like native T cells, are likely to trigger a GvH reaction. However, CAR-T cells are very toxic because they generate a cytokine release syndrome, the most severe grades of which can induce a cardiogenic shock or even respiratory distress. In addition, CAR-T cells are very expensive and time-consuming to produce.

It is thus necessary to develop efficient cell platforms that would be safer than conventional T cells. T cells with gamma delta TCR (γδ T cells or gd T cells) represent 5 to 10% of peripheral blood lymphocytes, of which 50 to 70% are composed of the subpopulation expressing γ9δ2 TCR. γδ T cells have the capacity to respond quickly after TCR engagement and to expand at the tumor site. Unlike αβT cells, γδT cells recognize antigens in an MHC-independent manner and detect abnormal cells through their danger signal molecular profile. Therefore these cells can be used therapeutically without triggering a GvH reaction. Their mechanism of action is based on their ability to destroy a target cell (tumor or infected) by triggering cell death via the release of perforin and granzyme and to recruit other immune cells via the release of interferon gamma (IFN γ) and interleukin 17 (IL-17). γδ T cells have been identified as the most favorable cells in terms of prognosis among the immune subpopulations infiltrated in 18,000 tumors, representing 39 tumor pathologies (Gentles et al., Nat. Med., 21:938-945, 2015).

Therapeutic uses of γδ T cells (TCR γ9δ2) were tested in some clinical trials on 559 patients with malignant blood diseases or solid tumors and 396 patients showed a measurable response (Feng & Zhou, Front. Immunol., 14, 2023). The different therapeutic strategies used were *in vivo* stimulation of γδ T cells and adoptive transfer of autologous or allogeneic γδ T cells, alone or combined with interleukin 2 (IL-2). Trials based on adoptive transfer showed good tolerance of γδ T cells with few adverse effects and an overall response rate of 35%.

In practice, in the cases of relapse of patients with malignant hematological diseases after allogeneic HSC transplantation, clinicians usually administer injections of fresh or thawed donor lymphocytes in order to enhance the graft versus leukemia effect (GvL). However, it must be taken into account that the administered product is not composed solely of pure T cells. Indeed, it is usually obtained either from leukapheresis or from a surplus of the HSC graft. In addition, among the T cells contained in the product, only a very small proportion are γδ T cells, the majority being αβ T cells likely to trigger a GvH reaction. This has the consequence of limiting the dose of T cells administered and therefore its effectiveness in the treatment of relapses.

In this context, there thus remains a genuine need for means allowing the production of a high amount of T cells enriched with the phenotype γδ.

The present invention is believed to meet such a need by providing a new method for expanding γδ T cells from mononuclear cells.

Unexpectedly, the Inventors discovered that sequential agitation during the first days of mononuclear cells culture allows the targeted amplification of the γδT cells subpopulation. Indeed, the Inventors showed that this step favors an enrichment of the circulating population present in the blood from healthy donors (from 0.5 to 5% depending on the individual) to reach a cell population composed almost exclusively of γδ T cells (> 80%) and almost devoid of αβ T cells (< 20%). This ability to selectively expand the γδ T cells subpopulation is highly advantageous in that it provides a high amount of γδ T cells, which then makes it possible to amplify γδ T cells on a larger scale within instrumented and controlled bioreactors. This method permits the production of a high quantity and quality of therapeutic products with good manufacturing practices (GMP).

In a main aspect, the present invention thus relates to a method for producing gamma delta (γδ) T cells comprising:
- a step (i) wherein mononuclear cells are incubated in presence of at least an activating compound selected from the group consisting of a phosphoantigen and a bisphosphonate, and
- a step (ii) wherein the mononuclear cells are cultured in a liquid culture medium comprising at least interleukin 2 (IL-2),
wherein step (ii) begins with a static phase and alternates static and agitated phases during at least 7 days.

As used herein, the term "T cell" (or "T lymphocyte") refers to an immune cell expressing CD3 (CD3+) and a T cell receptor (TCR+). T cells are important players in the adaptive arm (αβ subtype) and innate (αβ subtype) of the immune system. They respond to specific antigens presented by a major histocompatibility complex (MHC) dependent or independent manner.

As used herein, the term "γδ T cell" (or "gd T cell" or "gamma delta T cell") refers to a subset of T cells that express a distinct TCR, γδTCR, on their surface, composed of one γ-chain and one δ-chain. The predominant subset expresses a Vδ2 chain associated with a Vγ9 chain and represent 50 to 70% of the circulating γδ T cells in human adults, while a minor subset (approximately 30%) expresses a Vδ1 chain linked to a chain different from Vy9. In the present invention, the term "γδ T cells" includes, without limitation, all subsets of γδ T cells, in particular γ9δ2 T cells.

As used herein, the term "mononuclear cells" (or "peripheral blood mononuclear cells" or "PBMC") designates any blood cells having a round nucleus. These cells include lymphocytes (T cells, B cells, NK cells), monocytes and dendritic cells. While mononuclear cells are typically isolated from the peripheral circulation, in particular from buffy coat or leukapheresis samples, they can also be derived from bone marrow or cord blood.

As used herein, the term "culture medium" designates any medium appropriate for the culture of blood cells, in particular T cells. It includes, but is not limited to, RPMI 1640 medium, T vivo medium, CTS T cell culture media.

The step (i) allows the activation of γδ T cell subpopulation among the mononuclear cells by a boost with the activating compound.

The step (i) is preferably performed in static conditions to allow the adhesion of the accessory cells (other cells than γδ T cells) from the mononuclear cells at the bottom of the recipient.

As used herein, the term "phosphoantigen" refers to pyrophosphate-containing metabolites, which are recognized by γδ TCR and thus trigger the activation of γδ T cells. In particular, isopentenyl pyrophosphate (IPP) is generated from the endogenous mevalonate (MVA) pathway and accumulates intracellularly during dysregulated metabolism in many types of tumor cells. Also, (E)-4-hydroxy-3-methyl-but-2-enyl pyrophosphate (HMBPP) is a microbial metabolite from the isoprenoid pathway which represents "non-self" pathogen signals.

As used herein, the term "bisphosphonates" designates nitrogen-containing bisphosphonates, such as zoledronic acid or alkylamines, which cause intracellular IPP accumulation through inhibition of farnesyl pyrophosphate synthase.

The step (ii) allows the expansion and enrichment of the γδ T cell subpopulation.

As used herein, the term "static phase" means that no movement is applied to the culture.

The static phase allows the contact of γδ T cells with the accessory cells adhered at the bottom of the recipient.

As used herein, the term "agitated phase" means that at least one movement is applied to the cell culture. This term includes, with no limitation, stirring, shaking, rocking, rotating, shocking, agitating or inverting the recipient containing the cell culture.

The agitated phase allows the suspension of γδT cells, whereas the static phase allows the sedimentation of γδ T cells which results in their contact with accessory cells adhered to the bottom of the recipient and allows paracrine communications between cells.

In an embodiment, the invention relates to the method as defined above, wherein step (ii) begins with a static phase of 24 hours.

In an embodiment, the invention relates to the method as defined above, wherein step (ii) cyclically alternates static and agitated phases.

In an embodiment, the invention relates to the method as defined above, wherein step (ii) comprises at least one static phase and one agitated phase per day, preferably only one static phase and only one agitated phase per day during the first 7 days of culture.

In an embodiment, the invention relates to the method as defined above, wherein each agitated phase lasts at least 1 hour.

In an embodiment, the invention relates to the method as defined above, wherein each static phase lasts at least 23 hours.

In an embodiment, the invention relates to the method as defined above, wherein each static phase lasts 23 hours and each agitated phase lasts 1 hour.

In an embodiment, the invention relates to the method as defined above, wherein step (ii) begins with a static phase, alternates static and agitated phases during at least 7 days, and ends with an agitated phase.

In an embodiment, the invention relates to the method as defined above, wherein step (ii) comprises:
- a static phase of 24 hours, followed by
- a cycle of an agitated phase of 1 hour and a static phase of 23 hours during the next 7 days, optionally followed by
- an agitated phase during the next 14 days.

The agitation can be performed by using any device which prevents sedimentation of the cells in the culture medium.

In an embodiment, the invention relates to the method as defined above, wherein the agitated phases during step (ii) are carried out with an orbital shaker, a rocking shaker or a rotator shaker.

In an embodiment, the invention relates to the method as defined above, wherein the agitated phases are carried out at speed not exceeding 75 rotations per minute (rpm), preferably 70 rpm.

In an embodiment, the agitated phase is carried out at a speed selected from the group comprising 75, 70, 65, 60, 55, 50, 45, 40, 35, 30, 25, 20, 15, 10 and 5 rpm.

In an embodiment, the invention relates to the method as defined above, wherein the mononuclear cells have been obtained on peripheral blood.

In an embodiment, the invention relates to the method as defined above, wherein the mononuclear cells have been obtained by leukapheresis or buffy coat.

In an embodiment, the invention relates to the method as defined above, wherein the activating compound is a phosphoantigen, preferably selected from the group comprising isopentenyl pyrophosphate (IPP), ethyl pyrophosphate (EtPP) and hydroxy-methyl-butyl-pyrophosphate (HMBPP), more preferably IPP.

In an embodiment, the invention relates to the method as defined above, wherein the activating compound is a bisphosphonate, preferably zoledronic acid (Zometa).

In an embodiment, the invention relates to the method as defined above, wherein, during step (ii), the cells are maintained at a cell density not exceeding 2 × 10⁶ cells/ml.

In an embodiment, the invention relates to the method as defined above, wherein, among the population of cells obtained after step (ii), the population of CD3+ cells represents at least 75%, preferably 80%, of the total nucleated cells, and is composed of more than 70 % of γδ T cells.

In an embodiment, the invention relates to the method as defined above, further comprising:
- a step (iii), wherein the cells obtained from step (ii) are expanded in an agitated bioreactor under controlled conditions.

As used herein, the term "controlled conditions" means that the physicochemical parameters of the culture (e.g. pH, temperature, oxygen, carbon dioxide tension) are regulated.

In an embodiment, step (iii) is carried in an agitated bioreactor containing at least 1 liter of culture medium.

In an embodiment, step (iii) is carried in an agitated bioreactor containing from 1 to 10 liters of culture medium.

In an embodiment, the invention relates to the method as defined above, wherein, among the population of cells obtained after step (iii), the population of CD3+ cells represents at least 80 %, of γδT cells.

In an embodiment, the invention relates to the method as defined above, wherein the population of cells obtained after step (iii) comprises at least 80 % of γδ T cells.

In an embodiment, the invention relates to the method as defined above, wherein the population of cells obtained after step (iii) comprises at least 80 % of γδ T cells and is obtained in less than 22 days.

In an embodiment, the invention relates to the method as defined above, further comprising after step (iii):
- a step (iv), wherein the γδ T cells are recovered, and optionally sorted.

In an embodiment, the invention relates to the method as defined above, further comprising after step (iii):
- a step (iv), wherein the γδ T cells are recovered, and sorted if the population obtained after step (iii) still contains more than 20% of αβ T cells.

In a preferred embodiment, the invention relates to the method as defined above, which is carried out in fed-batch conditions.

In the present invention, the expression "fed-batch conditions" indicates that one or more nutrients is added continuously or intermittently into the medium after the start of cultivation.

In an embodiment, the invention relates to the method as defined above, wherein at least step (ii) or step (iii) is carried out in fed-batch conditions.

In an embodiment, the invention relates to the method as defined above, wherein both step (ii) and step (iii) are carried out in fed-batch conditions.

In another aspect, the invention relates to γδ T cells obtained by the method as defined above.

In an embodiment, the invention relates to the γδ T cells obtained by the method as defined above, wherein said γδ T cells mainly belong to the γ9δ2 subpopulation.

In an embodiment, the invention relates to the γδ T cells obtained by the method as defined above, wherein at least 75% of said γδ T cells belongs to the γ9δ2 subpopulation.

In an embodiment, the invention relates to the γδ T cells obtained by the method as defined above, wherein said γδ T cells are γ9δ2 T cells.

In an embodiment, the invention relates to the γδ T cells obtained by the method as defined above which are stored frozen.

In another aspect, the invention relates to γδ T cells obtained by the method as defined above, for use as a medicament.

In an embodiment, the medicament is an advanced therapeutic medicinal product (ATMP).

As used herein, the expression "advanced therapeutic medicinal product" (or ATMP) designates, but is not limited to, gene therapeutics, somatic cell therapeutics and tissue engineered products.

In an embodiment, the invention relates to γδ T cells obtained by the method as defined above, for use in an immunotherapy, preferably in the treatment or prevention of a cancer.

In an embodiment, said cancer is a leukemia, preferably an acute leukemia, most preferably a post-hematopoietic stem cell transplantation acute leukemia relapse.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

**Figure 1****.** Principle of PBMC collection from a buffy coat bag using the FICOLL density separation technique. Mononuclear cells rings are isolated after centrifugation. PBS (Phosphate Buffer Saline), LSM (Lymphocyte Separation Medium), PBMC (Peripheral Blood Mononuclear Cells).
**Figure 2****.** γδ T-cells sequential shaking program during expansion period and monitoring of cell characterization, expansion rate and biochemistry parameters.
**Figure 3****.** Flow cytometry gating strategy for analysis. This strategy assesses the percentage of cells of interest based on surface marker expression (CD3) and T-cell Receptor (TCR) expression (γδ or αβ).
**Figure 4****.** Comparative kinetics of surface marker expression during γδ T cell culture in static (black) and agitated (doted lines) conditions. Identity markers: CD3, αβTCR, γδTCR, CD16. Activation/exhaustion markers: DNAM-1, LAG-3, TIGIT, PD1.
**Figure 5****.** Growth kinetics of viable cells during the different phases of T cells culture. Cell expansion is expressed as total cells (concentration * volume), to avoid the dilution effects of regularly adding variable volumes of culture medium (fed batch, dotted arrows). The removal of 200 mL of culture indicates that the maximum reactor volume has been reached. The entire process was punctuated by daily sampling for process monitoring. Samples were taken at D0, D7, D13, D17, D21 and D27 for phenotypic analyses by flow cytometry.
**Figure 6****.** Phenotype characterization of expanded cells (D0-D2) by flow cytometry. The initial γδ T subpopulation of interest was significantly expanded through the culture and reached 90% of the total population at D27. αβ T cell subpopulation was significantly depleted in shaking culture conditions, compared to static culture (control culture).
**Figure 7****.** Growth kinetics of viable cells during the different phases of T cells culture for N=4 replicates. Cell expansion is expressed here as total viable cells quantity (concentration * volume), to avoid the dilution effects of on demands adding of optimal volumes of fresh supplemented culture media, L-Glutamine or glucose solution (fed batch mode), and daily withdrawals.
**Figure 8****.** Expansion factor of γδ T cells at Day 21 in static and in shaking cell culture. γδ T cells are significantly more expanded using shaking culture method than in static culture.
**Figure 9****.** Subpopulation repartition for static and shaking culture conditions. (A) Evolution of the percentage of γδ, αβ and other subpopulation of cells between Day 0 and Day 21 (harvest day) using either static or shaking culture conditions. γδ T cell subtypes repartition using static (B) and shaking (C) culture modes. N=4.
**Figure 10****.** Activation markers (NKG2D and DNAM-1) of γδ T cells. (A) Static culture conditions. (B) shaking culture conditions (N=5). (C) Comparison of both culture methods regarding activation markers expression between D0 and D21.
**Figure 11****.** Exhaustion markers of γδ T cells (PD-1, TIM-3, TIGIT). (A) Static culture conditions. (B) Shaking culture conditions N=5. (C) Comparison of both culture methods regarding exhaustion markers expression between D0 and D21.
**Figure 12****.** Kinetic of cell expansion in conventional mode of culture (static), in alternate shaking culture (shaken) and controls (T1: continuous shaking; T2: without feeder cells; T3: without Zometa pulse). Total cell quantity was determined by flow cytometry numeration.
**Figure 13****.** Evolution of the percentage of CD3+ cells (A), γδ and αβ T cells (B) depending on culture conditions (static, alternate shaking, continuous shaking (T1), without feeder cells (T2), without zometa pulse (T3)).

### EXAMPLES

### Materials & methods

### Culture media

RPMI medium 1640 (Fisher Scientific) supplemented with Human serum type AB (MALE) (SAB) at 10 % (V/V) (Sigma), Human IL-2 IS, premium gr. (200 µg) (Miltenyi Biotec) at 500 UI/mL, Antibiotic Antimycotic Solution (100×) (Sigma) at 1% (V/V) and L-Glutamine solution (200 mM) (Ref: Sigma) at 2% (V/V) is used. Media can punctually be supplemented by adding L-glutamine 200X (Sigma) and/or D-(+)-Glucose solution 45% (Sigma).

### Cells

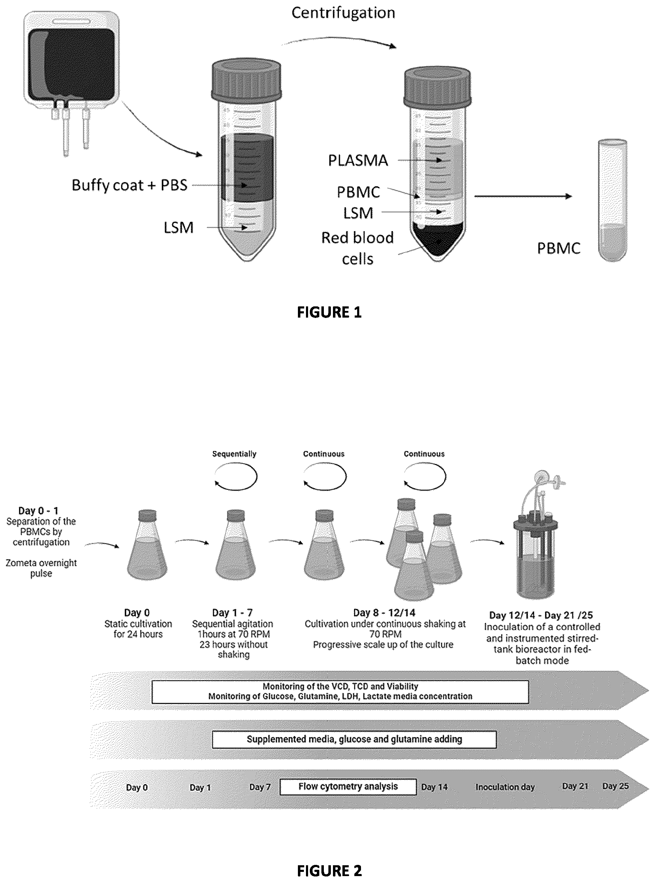

First, Peripheral Blood Mononuclear Cells (PBMC) are isolated from a buffy coat (Human volunteer adult donor, Etablissement Français du Sang (EFS)) using a density gradient centrifugation method. Buffy coat diluted with Phosphate Buffer Saline (PBS) (dilution factor: ½) is gently poured on a Lymphocyte Separation Medium (LSM) (Eurobio Scientific, France) cushion **(****Figure 1****).** The gradient density separation is performed by centrifugation (400 × g, room temperature, 20 min, Acceleration/break: 0). The PBMC ring is then transferred and washed with PBS (200 × g, room temperature, 20 min, Acceleration: 7, Break: 0). The PBMC pellet is resuspended in PBS and washed (400 × g, room temperature, 10 min, Acceleration: 9, Break: 9). The supernatant is discarded, and the cell pellet is finally resuspended in culture media. The volume is adjusted to reach a total cell concentration of 2.5×10⁶ cell.mL⁻¹

### Activation mechanism of γδ T-cells

After separation by centrifugation, PBMC are seeded at a concentration of 2.5 × 10⁶ viable cells mL⁻¹ in culture media Erlenmeyer flasks (total volume 125 mL, maximal working volume 25 mL). Zoledronic acid (ZOMETA, Novartis AG) (10µM) is added to cultures. Cells are incubated for 14 - 18h at 37°C, 80% H₂0, 5% CO₂ under static conditions. Cells are then washed by centrifugation (400 × g, room temperature, 10 min, Acceleration: 9, Break: 9) and resuspended in culture media at final concentration of 2.5×10⁶ viable cells mL⁻¹ in the same Erlenmeyer flasks, to keep adherent accessory cells for γδ T-cells activation.

### Culture conditions

Two expansion methods have been compared: one in static condition considered as gold standard (GS) and one in a shaking condition (SC).

γδ T cells expanded using GS conditions are cultivated for 21-25 days in ventilated in Erlenmeyer flasks (Day 0 - 7) to keep initial adherent accessory cells, and then in T-Flask (75cm²) after day 7. (37°C, 80% H₂0, 5% CO₂). Fresh media is added every 2 days in order to maintain the cell concentration between 1 - 2.5×10⁶ viable cells.mL⁻¹.

In comparison with GS cultures, cells in shaking conditions **(****Figure 2****)** are cultivated in Erlenmeyer flasks with ventilated lids in static conditions, during 24 hours. Then, a sequential shaking cycle is applied during 168 hours (Day 1 to day 8) with a cycle of 1 hour at 70 rpm an orbital shaking incubator (Kühner, LT-X LabTherm), and then followed by 23 hours of static culture conditions (Phase 1). This alternate regimen between shaking (1 hour) and static (23 hours) steps is performed during 7 days in order to allow either the physical contact between accessory and γδ T-cells or resuspension and homogenization during the agitated phase.

After 168 hours of culture (7 days), the cells in suspension (containing activated T-cells) are transferred in larger Erlenmeyer flasks to allow the culture scale-up, respecting a working volume/total volume of 1/5 ratio, optimal for gases exchanges. From day 8 until days 12/14, cells are expanded under continuous shaking condition (37°C, 80% H₂0, 5% CO₂, 70 rpm - 50mm) in an incubator (Kühner, LT-X LabTherm). This second phase (Phase 2) allows further enrichment in γδ T-cells and scale up. During both culture conditions (GS and SC), daily sampling is carried out under sterile conditions to evaluate VCD (Viable Cell Density), TCD (Total Cell Density) and cell viability using an automated cell counter based on the method of trypan blue exclusion (Vi-Cell XR - Beckmann Counter.) Biochemical characterisation of substrates (glutamine, glucose) and metabolic products (lactate, ammonium ions, lactate dehydrogenase (LDH)) is also carried out daily, using an automated biochemical analyzer (Gallery, ThermoFisher). Addition of fresh media or supplements (Glucose, L-Glutamine) is determined according to the cell density measurements in order to maintain cell concentration between 1 - 2.5 × 10⁶ viable cells mL⁻¹. Occasional additions of concentrated Glucose and L-Glutamine solution are performed to maintain at least 1g L⁻¹ of glucose, and 2 mM glutamine, to limit nutrient deprivations. During the expansion of γδ T-cells, both in agitated Erlenmeyer flasks and later in bioreactors, regular flow cytometry analyses are carried out to determine their phenotypic characteristics (see section below, *Cells characterization*)*.*

### Culture in bioreactor

Between day 12 and 14, depending on the cell density and γδ T-cell enrichment, a stirred-tank bioreactor with elephant ear mobile (pumping from bottom up) is inoculated (37°C, 50% O₂ relative to concentration in air, pH 7.20, 120 rpm). The quantity of γδ T-cells cells reached after phase 2 (total volume of flasks × cell concentration) is used to determine both the initial working volume and the date of the transfer of cells in the agitated bioreactor. To avoid any growth latency at the beginning of the culture in the bioreactor, a minimum concentration of γδ T-cells is required (2 × 10⁶ cells mL⁻¹) for a 1 L bioreactor. The pH and pO₂ values are regulated using the Air/Nitrogen and CO₂/NaOH (0.1 M) regulator pairs respectively. During the expansion of T cells in the agitated bioreactor, the GS culture is still maintained in static conditions (See section *Culture conditions*)*.* Daily sterile sampling is carried out to monitor VCD, TCD, cell's viability and biochemical parameters (Glucose, Glutamine, Lactate, LDH) using both cell counter (ViCell XR, Beckman) and automated biochemical analysers (Gallery, Thermo). Cells are characterized by flow cytometry right after bioreactor's inoculation (Day 12 -14), and at day 21 (end of the expansion process).

### Cells characterization

Cells from GS and SC methods are characterized by flow cytometry (MACSQuant analyzer, Miltenyi Biotec). Identity, but also activating and exhaustion markers are monitored at day 0, day 7, bioreactor inoculation day, day 14, day 21 and day 25/end of the culture) using two different cytometry multiple-markers-panels of antibodies **(Table 1).**

**Table 1: Identity and activity/exhaustion flow cytometry panel markers for γδ T-cells phenotypic characterization during expansion processes in static and agitated conditions (Identity = to identify cells as LT and also to differentiate between αβ, and γδ sub-phenotype; Activation = to ensure that γδ T-cells are functional; Exhaustion = Cell exhaustion related to culture time, or cytokine boost).**

| **Identity Panel** | **Activity/exhaustion panel** |
|---|---|
| CD3-APCVio770 (Miltenyibiotec) | |
| TCRγδ-BV423 (Clone 11F2) (Miltenyibiotec) | |
| TCRγδ-BV423 (Clone REA 591) (Miltenyibiotec) | |
| TCR α/β Antibody, anti-human, FITC, REAfinity^{™} (Miltenyibiotec) | CD279 (PD1) Antibody, anti-human, Vio ^{®} Bright FITC (Miltenyibiotec) |
| TCR Vδ2 Antibody, anti-human, PE, REAfinity^{™} (Miltenyibiotec) | TIGIT Antibody, anti-human, PE, REAfinity^{™} (Miltenyibiotec) |
| CD45RA Antibody, anti-human, APC, REAfinity^{™} (Miltenyibiotec) | CD366 (TIM-3) Antibody, anti-human, PE-Vio ^{®} 770, REAfinity^{™} (Miltenyibiotec) |
| CD16 Antibody, anti-human, PE-Vio ^{®}770, REAfinity^{™} (Miltenyibiotec) | CD226 (DNAM-1) Antibody, anti-human, APC, REAfinity^{™} (Miltenyibiotec) |
| Hu CCR7 (CD197) BV510 3D12 50Tst (BD Biosciences) | Hu CD314 (NKG2D) BV510 1D11 50 Tst (BD Biosciences) |
| 7AAD Staining solution (Miltenyibiotec) | |

For γδ T cell enrichment follow-up, flow cytometry analyses are performed weekly between day 0 and day 21. The "Identity" panel was designed to follow γδ and αβ T cell sub-populations during expansion. Within the γδ population, cells expressing a TCR with a δ2 chain are particularly followed as well as cells with a naive, central memory or effector memory phenotype based on CCR7 and CD45RA expression. The γδ T cell exhaustion is followed using PD1 (programmed cell death protein 1), TIM-3 and TIGIT. High levels of these three proteins generally result in an exhaustion as well as a loss of LT functions. Regarding activating receptor expression, DNAM-1 is an activating molecule that promotes tumor cell lysis through its interaction with the PVR/CD155 (Polioma Virus Receptor) protein expressed on the surface of tumor target cells. The DNAM-1/PVR pair is thus involved in tumor and viral immunosurveillance. NKG2D recognizes ULBP and MICA/B molecules expressed on leukemia cells. 7-Aminoactinomycin D (7-AAD) was also added to the sample a few minutes before analysis to check cell viability.

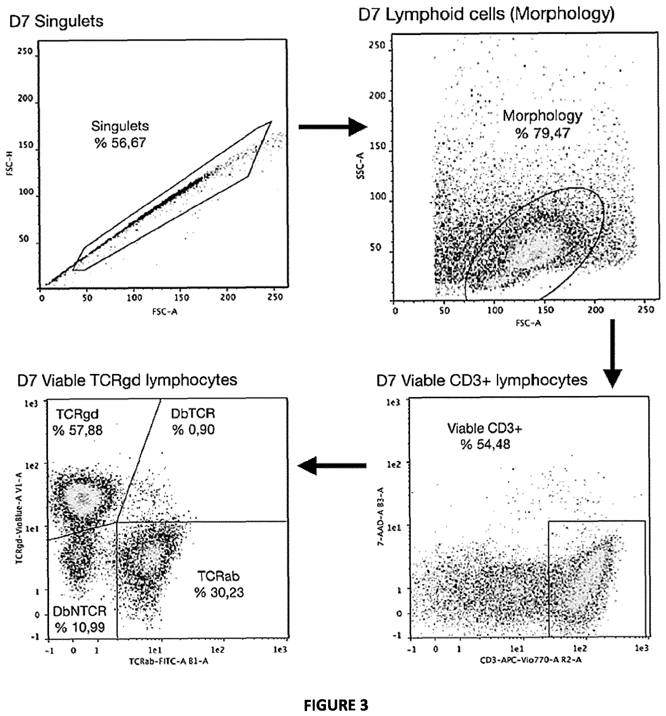

The gating strategy implemented for γδ T-cell phenotyping is shown in **Figure 3****.** Cells are visualized by labelling with anti-CD3 antibody PEVio-770A and by adding 7AAD to the sample. CD3+ /7AAD-cells correspond to the viable T lymphocytes. Finally, γδ or αβ TCR expression was determined after gating on CD3+/7AAD- cells. The expression of the markers belonging to the 2 panels were then determined on CD3+/7AAD- / TCR γδ+ cells.

### Example 1. γδ T cell expansion in sequential shaking conditions

The method of the invention aims to expand clinical-grade T γδ cells intended for the treatment of post-transplant leukemia relapse. To achieve this goal, the culture method needs to specifically amplify from the PBMC the γδ phenotypic subpopulation of T lymphocytes, which is only a scarce population in healthy donor's blood (between 0.5 and 5% depending on the individuals).

A selective amplification should allow to obtain a high enrichment (above 80%) of γδ T cells and, at the same time, an efficient depletion (below 15%) of the αβ T cell subpopulation responsible for the GvH reaction. Also, a selective amplification should produce a high quantity of γδ T cells rendering possible a culture of γδ T cells in controlled bioreactors, at a scale up to 5-10 liters. In addition, it is necessary to limit the cell "exhaustion", which can result in a therapeutic product with little or no activity.

In the present invention, it was hypothesized that a sequential agitation during the first step of the culture could improve gas and nutritional transfers while keeping a contact with the accessory cells adhered at the bottom of the flasks.

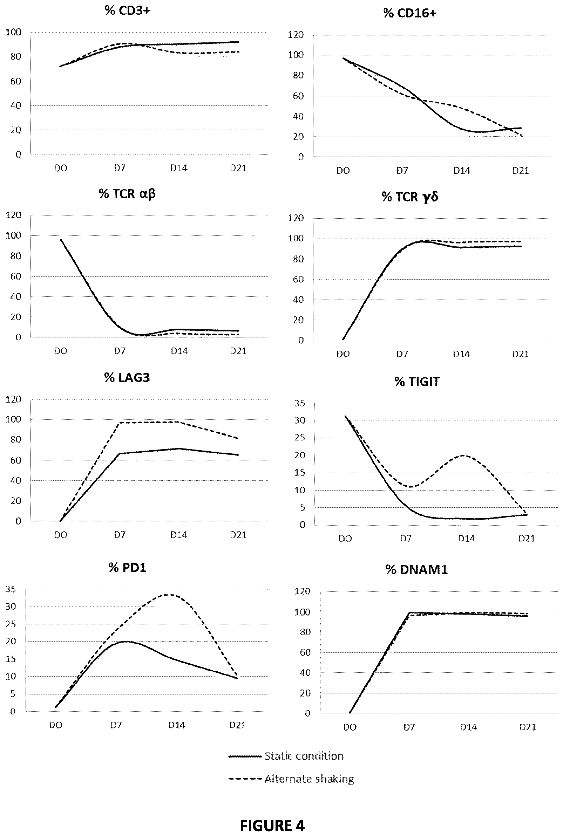

For this purpose, PBMC were isolated using a Ficoll gradient, treated with Zometa (zoledronic acid), and seeded in static conditions (control) and in shaking conditions (with intermittent agitation in shaking flasks). An initial cytometry analysis was performed at D0 to evaluate surface markers. The same analyses were performed at D7, D14 and D21. Weekly sample collections were performed for cell counting and phenotyping analyses. The kinetic expression of each target marker during the culture, in both shaking and static conditions, is shown in **Figure 4****.**

Specific surface markers related to the identity of cells in suspension, such as CD3, TCR γδ, TCRαβ and CD16 were quantified. With regard to the purity of cultures, 71% CD3+ lymphocytes at D0 have been measured, and reached up to 91% and 83% in static and shaking conditions respectively at D21. More specifically, 92% and 96% of cells expressed γδ TCR in static and in agitated conditions respectively, with a multiplication rate higher than 800x from D0 to D21.

With regard to exhaustion markers, LAG3 increased for both culture conditions until D7 and then stabilized until the end of the culture. Maximum in PD-1 expression was reached at D14 and then declined until the end of culture. This increase in cell expression was more pronounced in shaking cultures compare to static cultures (33 vs. 20% respectively). TIGIT+ cells decreased completely during static culture whereas this marker expression was maintained in shaking conditions until D14, then decreased after a peak. At D21, expression of this marker is completely abolished in both culture conditions. Interestingly, a strong induction in DNAM-1 expression was observed in almost 100% of cells after D7 and until the end of the culture, whatever the cell culture conditions, indicating gain in cytotoxic activity and therefore an interesting functionality of cells.

The experiments carried out in both static and agitated conditions showed that cells had a strong proliferative capacity. Additionally, exhaustion markers such as PD-1 and TIGIT were lost during the culture whereas activation markers (i.e. DNAM-1) were strongly induced. Only the agitated condition allows to reach enough γδ cells to seed the bioreactor used for the next step of expansion.

### Example 2. y6 T cell expansion in sequential shaking conditions and passage in controlled bioreactors

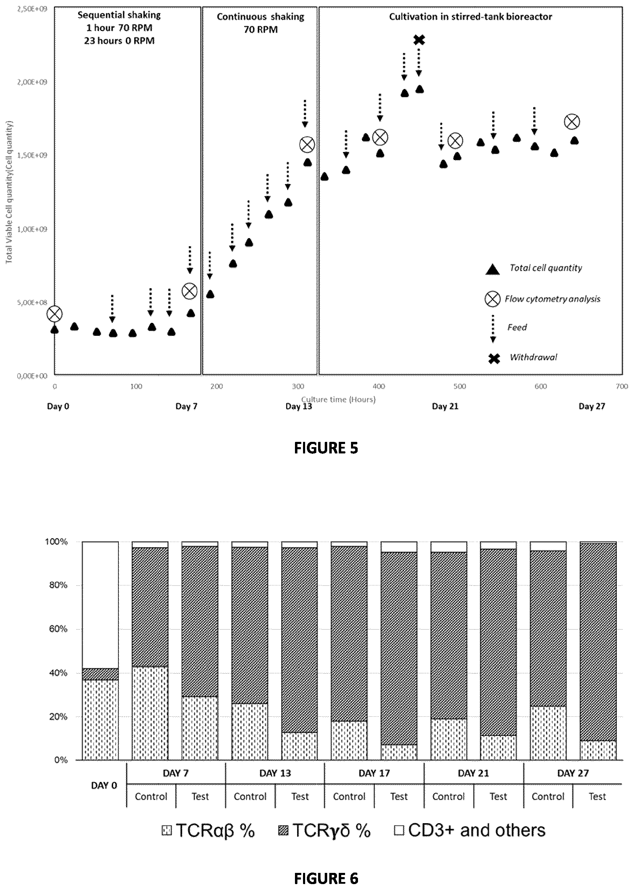

In order to confirm its efficiency and robustness despite biological variability, the shaking process was carried out using independent donor buffy coats. The following experiment generated a reference growth kinetic (Figure 5), that was used to orient further replicate in terms of cell sampling and phenotyping using flow cytometry, or in terms of feeding and cell harvesting timing.

In addition, complete culture medium (fed batch mode) was added at regular time intervals from day 3, depending on cell growth and residual glucose and glutamine concentration in culture media, to maintain a sufficient amount of nutrients in the bioreactors.

Moreover, flow cytometry analyses of cells confirmed the progressive and substantial enrichment in γδ T cells (Figure 6). Across all checkpoints (D0, D7, D13, D17, D21 and D28), γδ T cells identity markers indicated an increase in purity up to 97.65% of CD3+ cells (T cells identity), including 90% of γδ T cells among them at D27.

Compared with the control experiment (static culture), the total expansion rate increased 76-fold (vs. 3-fold for the control), with a 1,600-fold enrichment in γδ T cells (vs. 34-fold for the control), while αβ T cells were significantly reduced, with less than 10% of T cells in the agitated bioreactor vs. more than 25% in the static control culture.

The cell identity and activation markers measured (CD3 positive at D21 = 96.96% and DNAM-1 positive at D27 = 99.45%), indicate activation strong expression, whereas cell exhaustion markers such as PD-1+ at D27 remained low (less than 2.5%).

Repetition of experiments were performed to confirm results and trends, using the method combining sequential shaking program during the first phase. All replicates (using different sex, age and blood group donor buffy coats) led to similar trends, despite variations in initial cell quantities and purities, and consequently leading to variable final amount of γδ T cells after expansion.

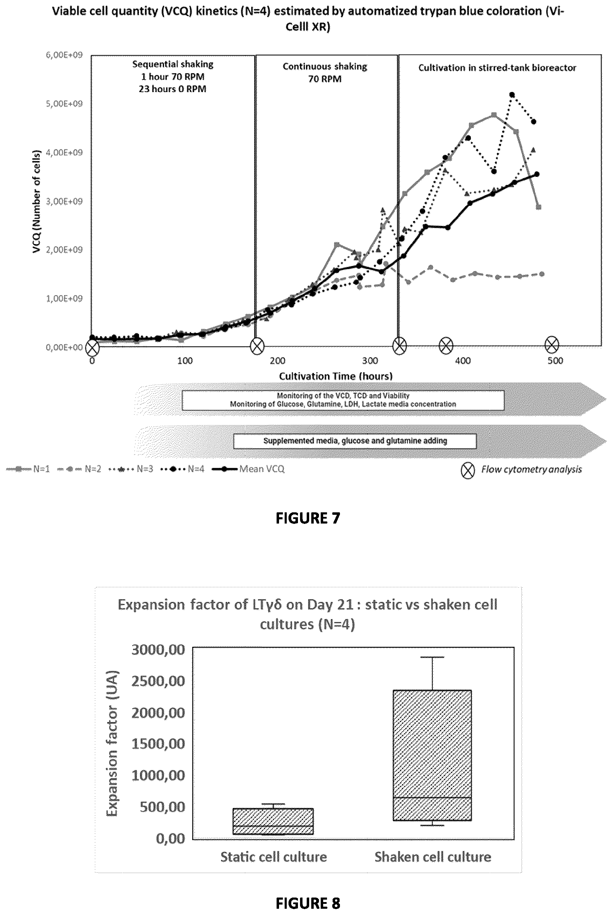

In four described replicates (N=1; N=2; N=3; N=4), the cell initial quantity has been significantly expanded **(****Figure 7****).** γδ T cells were significantly more expanded in shaking culture conditions than in static culture conditions **(****Figure 8****).**

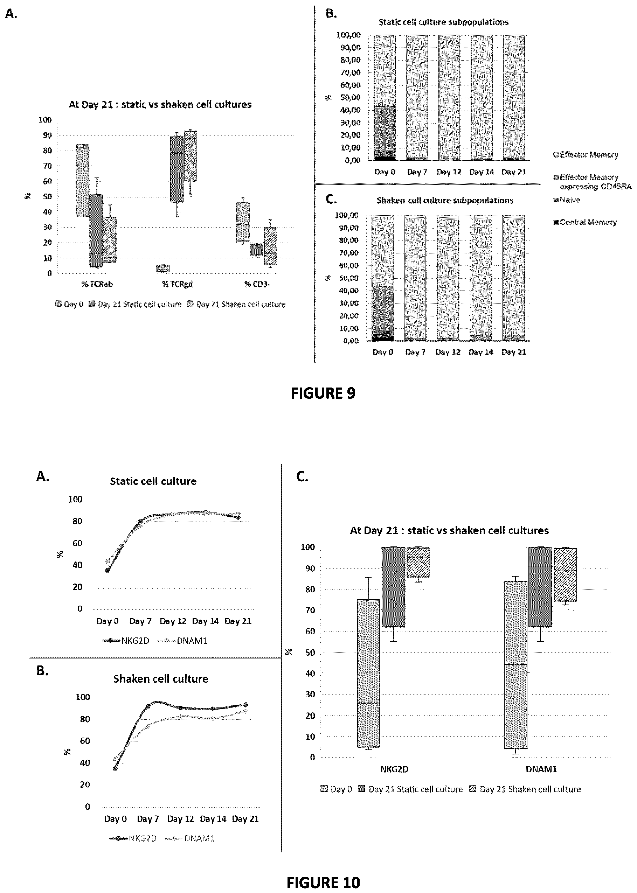

Variation in subpopulations in static and shaking culture conditions displayed the same pattern **(****Figure 9****, A),** with a significant depletion in CD3 negative cells. The αβ T cells, initially predominant at day 0, are efficiently depleted at day 21 in both culture conditions (bellow 15 % of the cells). γδ T cells, are significantly enriched at day 21 compared to the beginning of the culture and reached approximately 80% in static conditions, and near 90% in bioreactor cultures. Experiments showed a better purity of the final product in bioreactor cultures compared to conventional static culture. In each experiment, more than 80% of cells were of γδ T cells whereas αβ T cells remained below 15%. In addition, other γδ T phenotype markers were similar whatever the culture conditions **(****Figure 9****, B and C).**

Cells purity and subtypes repartition combined with a high expansion rate, resulted in higher quantity of cells of γδ T cells Effector memory (TEM) obtained in bioreactor compared to static culture.

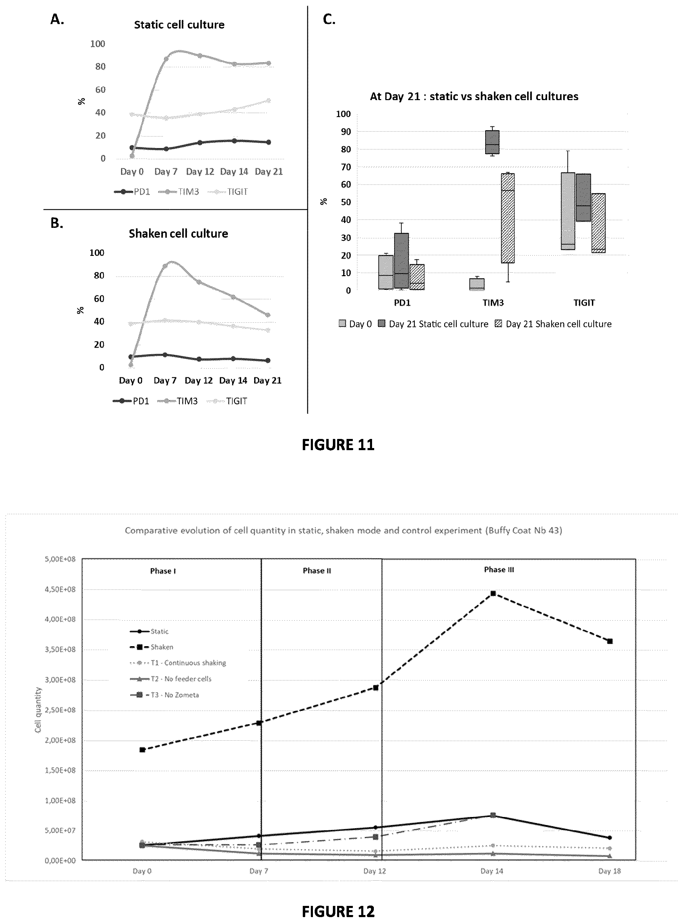

Other markers such as those related to activation and exhaustion were monitored by flow cytometry **(****Figure 10****, A and B).** Activation markers (NKG2D, and DNAM1) appeared not to be affected by the cultivation method suggesting that after expansion, the functionality, and consequently their therapeutic properties were maintained. Major cons of cells expansion in bioreactor could be an earlier cell exhaustion. However, bioreactor cultures exhibited lower level of TIM 3, PD1 and TIGIT exhaustion markers expression at the end of the process **(****Figure 11****, B),** compared to static cultures **(****Figure 11****, A).** Despite the mechanical stress potentially induced by the agitation in a bioreactor, an efficient expansion of γδ T cells (CD3+, γδ TCR+, Vδ2 +), displaying a good activation **(****Figure 10****, C)** and a low exhaustion **(****Figure 11****, C)** was obtained.

### Example 3. Further experiments with various controls

In order to demonstrate the efficiency of the method of the invention, based on a sequential shaking culture, different experiments have been carried out.

Starting from a single buffy coat, five different culture conditions experiments have been carried out (n=2): (i) conventional static culture in flasks, (ii) sequential shaking culture (as described above) and (iii) three control cultures : with continuous shaking during the three phases (T1), (iv) with sequential shaking (phase I) but without feeder cells (T2) and (v) with sequential shaking, including feeder cells but without Zometa (T3).

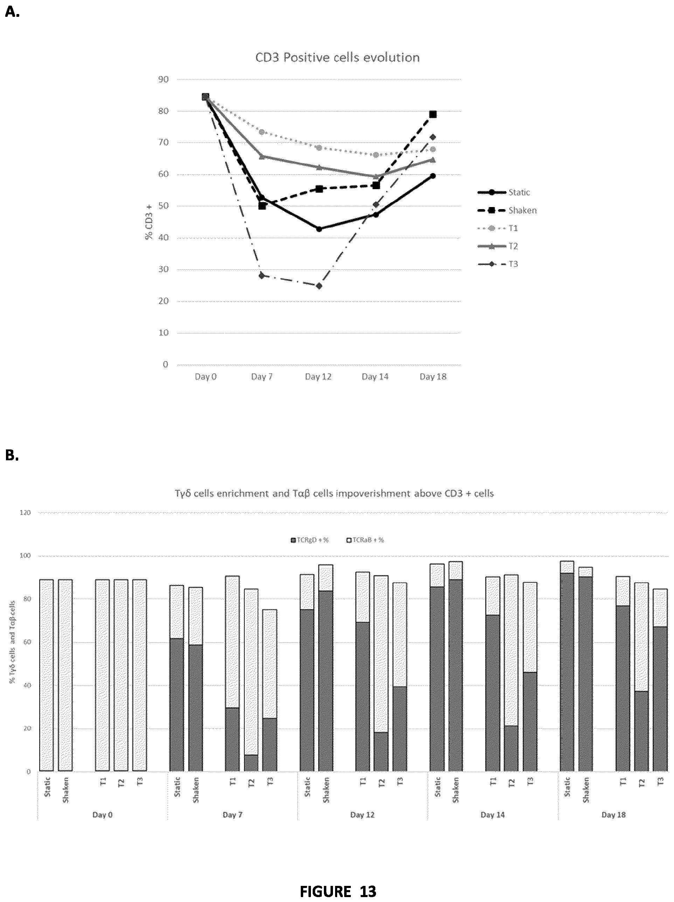

Comparison of cell quantity kinetics between D0-D18 confirmed the cell expansion efficiency using the sequential shaking conditions **(****Figure 12****).** Conventional static culture, and controls (T1, T2, T3) exhibited similar poor cell expansion, indicating that sequential shaking of cells, preliminary treatment with Zometa and the presence of adherent feeder cells are essential to promote total cell expansion. Taking into account these results, the γδ T cells enrichment as well as the αβ T cells depletion were also monitored by flow cytometry **(****Figure 13****).**

Starting from a single buffy coat, initial CD3 + cells in each culture conditions were equivalent (84 %), then decreased at day 18, 79% of the cell in shaken condition are CD3 + cells, compared to 59 % in static conditions and 67,83%, 64,7 % and 71,7 % for T1, T2, T3 respectively. Within the CD3 + cells, the subpopulation percentages of cells of interest (γδ TCR + cells) and of αβ TCR+ cells were compared. Both static and shaking conditions resulted in are similar enrichment in γδ TCR+ cells with 91,87 % and 90,215 % respectively and in depletion in αβTCR + cells, with 5,63% and 4,43 % respectively. As expected, controls (T1, T2, T3) exhibited lowered enrichment in γδ T cells and depletion in Tαβ cells, during the cultures **(****Figure 13****).** Taking together, these results **(****Figures 12** **and** **13****)** confirm the poor performance of the expansion process in continuous shaking, without feeder cells and without Zometa, but also the superiority of the sequential shaking process compared to conventional static method in terms of γδ T cells quantity after expansion.

## Claims

1. A method for producing gamma delta (γδ) T cells comprising:
- a step (i) wherein mononuclear cells are incubated in presence of at least one activating compound selected from the group consisting of a phosphoantigen and a bisphosphonate, and
- a step (ii) wherein the mononuclear cells are cultured in a liquid culture medium comprising at least interleukin 2 (IL-2),
wherein step (ii) begins with a static phase and alternates static and agitated phases during at least 7 days.

2. The method according to claim 1, wherein step (ii) cyclically alternates static and agitated phases.

3. The method according to claim 1 or 2, wherein step (ii) comprises at least one static phase and one agitated phase per day, preferably only one static phase and only one agitated phase per day during the first 7 days of culture.

4. The method according to any one of claims 1 to 3, wherein each static phase lasts 23 hours and each agitated phase lasts 1 hour.

5. The method according to any one of claims 1 to 4, wherein step (ii) begins with a static phase of 24 hours.

6. The method according to any one of claims 1 to 5, wherein step (ii) comprises:
- a static phase of 24 hours, followed by
- a cycle of an agitated phase of 1 hour and a static phase of 23 hours during the next 7 days, optionally followed by
- an agitated phase during the next 14 days.

7. The method according to any one of claims 1 to 6, wherein the agitated phases during step (ii) are carried out with an orbital shaker, a rocking shaker or a rotator shaker.

8. The method according to any one of claims 1 to 7, wherein the mononuclear cells have been obtained on peripheral blood, in particular by leukapheresis or buffy coat.

9. The method according to any one of claims 1 to 8, wherein the activating compound is a phosphoantigen selected from the group comprising isopentenyl pyrophosphate (IPP), ethyl pyrophosphate (EtPP), hydroxy-methyl-butyl-pyrophosphate (HMBPP), more preferably IPP.

10. The method according to any one of claims 1 to 9, wherein the activating compound is a bisphosphonate, preferably zoledronic acid (Zometa).

11. The method according to any one of claims 1 to 10, wherein, during step (ii), the cells are maintained at a cell density not exceeding 2 × 10⁶ cells/ml.

12. The method according to any one of claims 1 to 11, further comprising:
- a step (iii), wherein the cells obtained from step (ii) are expanded in an agitated bioreactor under controlled conditions.

13. The method according to any one of claims 1 to 12, further comprising after step (iii):
- a step (iv), wherein the γδ T cells are recovered, and optionally sorted.

14. γδ T cells obtained by the method as defined in any one of claims 1 to 13.

15. γδ T cells obtained by the method as defined in any one of claims 1 to 13, for use in an immunotherapy, preferably in the treatment or prevention of a cancer.
